Europäisches Patentamt

(19) European Patent Office

Office européen des brevets

(11) Publication number : 0 457 527 A1

(12) **EUROPEAN PATENT APPLICATION**

(21) Application number : 91304271.9

(22) Date of filing : 13.05.91

(51) Int. Cl.⁵ : **C12N 15/85, C12N 15/58, C07K 15/00**

(30) Priority : 14.05.90 JP 123573/90
29.03.91 JP 93105/91

(43) Date of publication of application :
21.11.91 Bulletin 91/47

(84) Designated Contracting States :
**BE CH DE DK ES FR GB IT LI NL SE**

(71) Applicant : **THE GREEN CROSS CORPORATION**
**3-3, Imabashi 1-chome Chuo-ku**
**Osaka-shi Osaka (JP)**

(72) Inventor : **Tanabe, Toshizumi, Chuo Kenkyusho, The Green**
**Cross Corporation, 1180-1, Shodai Otani-2-chome**
**Hirakata-shi (JP)**
Inventor : **Amatsuji, Yasuo, Chuo Kenkyusho, The Green**
**Cross Corporation, 1180-1, Shodai Otani-2-chome**
**Hirakata-shi (JP)**
Inventor : **Morita, Masanori, Chuo Kenkyusho, The Green**
**Cross Corporation, 1180-1, Shodai Otani-2-chome**
**Hirakata-shi (JP)**
Inventor : **Hirose, Masaaki, Chuo Kenkyusho, The Green**
**Cross Corporation, 1180-1, Shodai Otani-2-chome**
**Hirakata-shi (JP)**

Inventor : **Nakajima, Masahide, Chuo Kenkyusho, The Green**
**Cross Corporation, 1180-1, Shodai Otani-2-chome**
**Hirakata-shi (JP)**
Inventor : **Nakagawa, Yukimitsu, Chuo Kenkyusho, The Green**
**Cross Corporation, 1180-1, Shodai Otani-2-chome**
**Hirakata-shi (JP)**
Inventor : **Hayasuke, Naofumi, Chuo Kenkyusho, The Green**
**Cross Corporation, 1180-1, Shodai Otani-2-chome**
**Hirakata-shi (JP)**
Inventor : **Kawabe, Haruhide, Chuo Kenkyusho, The Green**
**Cross Corporation, 1180-1, Shodai Otani-2-chome**
**Hirakata-shi (JP)**
Inventor : **Yamauchi, Takeshi, Chuo Kenkyusho, The Green**
**Cross Corporation, 1180-1, Shodai Otani-2-chome**
**Hirakata-shi (JP)**
Inventor : **Asakura, Eiji, Chuo Kenkyusho, The Green**
**Cross Corporation, 1180-1, Shodai Otani-2-chome**
**Hirakata-shi (JP)**
Inventor : **Uno, Shuhsei, Chuo Kenkyusho, The Green**
**Cross Corporation, 1180-1, Shodai Otani-2-chome**
**Hirakata-shi (JP)**
Inventor : **Kamiya, Toshio, Chuo Kenkyusho, The Green**
**Cross Corporation, 1180-1, Shodai Otani-2-chome**
**Hirakata-shi (JP)**

(74) Representative : **Coleiro, Raymond et al**
**MEWBURN ELLIS 2 Cursitor Street**
**London EC4A 1BQ (GB)**

(54) Plasmids, transformed animal cells and process for producing foreign protein.

(57)    A plasmid for enhancing expression in an animal cell comprises (i) a promoter capable of regulating the expression of a foreign protein in an animal cell and (ii) a DNA encoding dihydrofolate reductase (DHFR) upstream of which an urokinase (UK) promoter is ligated. The plasmid may also additionally comprise the DNA encoding the foreign protein. These plasmids are useful for the mass production of a foreign protein such as plasminogen activator, in an animal cell by genetic engineering.

EP 0 457 527 A1

EP 0 457 527 A1

## BACKGROUND OF THE INVENTION

### Field of the Invention

The present invention relates to a DNA encoding dihydrofolate reductase (DHFR) under the control of an urokinase (UK) promoter and a DNA encoding a foreign protein upstream of which a promoter capable of regulating the expression of the protein in an animal cell is ligated, an animal cell transformed with the plasmid, and relates to a process for producing the foreign protein which comprises culturing the animal cell to express the foreign protein.

### Related Art Statement

As for a host for producing a desired physiologically active substance by genetic engineering technology, investigations have been firstly focused on E. coli and then shifted to microorganisms such as yeast and Bacillus subtilis which may be cultivated by simple procedures, and host-vector systems utilizing such microorganisms have been intensively developed [Goeddel, D.V., Itakura, K., et al., Proc. Nalt. Acad. Sci. U.S.A., 76, l06 (l979); and Nagata, S., Taira, S.H. and Weissmann, C., Nature, 284, l3l6 (l980)].

In recent years, however, attention has been brought to an expression system using an animal cell as a host for producing a natural type high molecular glycoprotein in a soluble form by genetic engineering technology.

An example of expression in an animal cell is disclosed in Japanese Patent Application Laid-Open No. 6l-l77987.

For enhancing expression in an animal cell, a technique for amplification with a DNA encoding DHFR has been proposed. A potent promoter should be chosen to express a cloned gene as highly efficiently as possible. At first, a technique has been adopted to regulate a DNA encoding DHFR using simian virus (SV40) promoter (Japanese Patent Application Laid-Open No. 63-l05975), but it has been recently proposed to regulate a DNA encoding DHFR using DHFR promoter (Japanese Patent Application Laid-Open No. 63-27348l).

In order to enhance the degree of the expression of a recombinant protein to produce it in large quantities, it is thus desired that an expression vector have a promoter which can achieve the transcription of a foreign gene encoding the protein in a high efficiency.

## SUMMARY OF THE INVENTION

In view of the foregoing situations, the present inventors have made various investigations. As a result, it has been found that by using UK promoter as a promoter for regulating a DNA encoding DHFR, the degree of amplification is much superior to the level conventionally known. The present invention has thus been accomplished. That is, the present invention is concerned with:

(l) A plasmid (hereinafter referred to as plasmid① ) having a promoter capable of regulating the expression of a foreign protein in an animal cell and a DNA encoding dihydrofolate reductase (DHFR) upstream of which an urokinase (UK) promoter is ligated;

(2) A plasmid (hereinafter referred to as plasmid② ) having a DNA encoding a foreign protein upstream of which a promoter capable of regulating the expression of the protein in an animal cell is ligated and a DNA encoding dihydrofolate reductase (DHFR) upstream of which an urokinase (UK) promoter is ligated;

(3) An animal cell transformed with the plasmid② ;

(4) An animal cell cotransformed with a plasmid (hereinafter referred to as plasmid③ ) having a DNA encoding a foreign protein upstream of which a promoter capable of regulating the expression of the protein in an animal cell and another plasmid (hereinafter referred to as plasmid④ ) having a DNA encoding dihydrofolate reductase (DHFR) upstream of which an urokinase(UK) promoter is ligated;

(5) A process for producing a foreign protein which comprises culturing an animal cell of (3) above to express the foreign protein and recovering it; and

(6) A process for producing a foreign protein which comprises culturing an animal cell of (4) above to express the foreign protein and recovering it.

## BRIEF DESCRIPTION OF THE DRAWINGS

Fig. l shows procedures for constructing pTT03 and pHR24.
Fig. 2 shows the structures of pUH3 and pUH7 which are starting plasmids for constructing pTT03.
Fig. 3 shows the structure of subclone pUKPl.

2

EP 0 457 527 A1

Fig. 4 shows procedures for constructing pTT06
Fig. 5 shows procedures for constructing pTT08 and pTT09.
Fig. 6 shows the structures of pTT08 and pTT09.
Fig. 7 shows an amount of Asn[24]-PPA produced in MTX-resistant cell.
Fig. 8 shows the steps of constructing plasmids (pTY007 and pTY008) for expressing ATIII.
Fig. 9 shows gene bearing pTY007 and its restriction enzyme map, wherein the symbols denote the following:

l: SV40-derived enhancer/promoter
2: human ATIII cDNA
3: SV40 splicing junction and polyadenylation signal
4: human urokinase promoter
5: mouse dhfr cDNA
6: SV40 splicing junction and polyadenylation signal
7: Amp'

Fig. l0 shows distribution of ATIII-producing clones in CHO cells transfected with pTY007 and pTY008.

DETAILED DESCRIPTION OF PREFERRED EMBODIMENTS

Hereafter the plasmid, transformed animal cells and the process for producing the foreign protein are described in detail.

[I] Plasmid①

In the present invention, plasmid① comprises:
(i) a promoter capable of regulating the expression of a foreign protein in an animal cell, and
(ii) a DNA encoding dihydrofolate reductase (DHFR) upstream of which an urokinase (UK) promoter is ligated.

By incorporating into the plasmid① a DNA encoding a foreign protein upstream of the promoter capable of regulating the expression of the protein in an animal cell, an expression vector can be constructed for expressing the foreign protein in an animal cell. Hence, the plasmid① can be used as a unit for constructing the expression vector.

The promoter capable of regulating the expression in an animal cell is derived from polyoma, adenovirus 2 or simian virus 40 (SV40) which has been used most widely. The early and late promoters of SV40 virus are particularly preferred. This is because these promoters can be readily obtained as fragments containing an origin of replication in SV40 [Fiers et al., Nature, 273, ll3 (l978)]. A fragment containing about 250 bp region from Hind III site to Bgl I site in the origin of replication may also be used. In addition, promoters and enhancers associated with a gene encoding a foreign protein may also be used so long as they are compatible with a host cell.

As the promoter/enhancer used in an expression vector in an animal cell, there may be used promoter/enhancer of SV40 early gene or late gene, adenovirus major late promoter domain, globulin enhancer/promoter domain, LTR of RNA virus, metallothionein promoter domain and β-actin promoter. As an origin of replication, those derived from SV40 or other viruses (e.g., polyoma, adenovirus, VSV and BPV) may be incorporated into an expression vector. An automonous replication system of a host cell chromosome may also be used. If an expression vector is integrated into the chromosome of a host cell, it may be sufficient to use the autonomous replication system.

The DNA encoding DHFR upstream of which UK promoter is ligated is so designed that the DNA encoding DHFR is operative under the control of UK promoter. UK promoter and the DNA encoding DHFR are both known and disclosed in Nucl. Acids. Res., l3, 2759-277l (l985) and in Japanese Patent Application Laid-Open Nos. 59-l92089 and 63-l05675, respectively.

The plasmid① may also contain an origin of replication upstream of the promoter capable of regulating the expression of a foreign protein in an animal cell, and ribosome binding site, RNA splicing site, poly A addition site or transcription termination sequence downstream of the promoter.

[II] Plasmid②

In the present invention, plasmid② comprises:
(i) a DNA encoding a foreign protein upstream of which a promoter capable of regulating the expression of the protein in an animal cells is ligated; and

3

(ii) a DNA encoding dihydrofolate reductase (DHFR) upstream of which an urokinase (UK) promoter is ligated.

In the plasmid②, the promoter capable of regulating the expression of a foreign protein in an animal cell is ligated with the DNA encoding a foreign protein at the upstream thereof. Thus, the DNA encoding a foreign protein is operative under the control of the promoter.

Examples of a DNA encoding a foreign protein include DNA sequences encoding pro UK (Japanese patent Application Laid-Open No. 60-180591), modified pro UK (Japanese patent Application Laid-Open Nos. 63-146789, 1-126433 and 1-126434), t-PA (Japanese Patent Application Laid-Open Nos. 59-183693 and 59-42321), HBsAg (Japanese Patent Application Laid-Open No. 59-36698), PreS-HBsAg (Japanese Patent Application Laid-Open No. 56-63995), IFN-γ (Japanese Patent Application Laid-Open No. 61-108397), AT III [Japanese Patent Application Laid-Open No. 58-162529, Susan Clark Bock et al., Nucleic Acids Res., 10, 8113-8125 (1982)] and CSF (Japanese Patent Application Laid-Open No. 61-199787). DNA sequences encoding active fragments or derivatives of these foreign proteins may also be used.

In this plasmid②, the unit composed of the promoter capable of regulating the expression of a foreign protein in an animal cell and a DNA encoding a foreign protein and the unit composed of UK promoter and DNA encoding DHFR may be arranged in the same direction or may be arranged in the inverted direction. The plasmid② may also contain an origin of replication upstream of the DNA unit encoding a foreign protein, downstream of which ribosome binding site, RNA splicing site, poly A addition site or transcription termination sequence may be ligated.

## [III] Plasmids③ and④

In the present invention, plasmid③ comprises a DNA encoding a foreign protein upstream of which a promoter capable of regulating the expression of the protein in an animal cell is ligated.

Plasmid④ comprises a DNA encoding dihydrofolate reductase (DHFR) upstream of which an urokinase (UK) promoter is ligated.

These plasmids③ and④ carry the same DNAs as the DNA components (i) and (ii) of plasmid② described above, respectively. Plasmids③ and④ are used to cotransform an animal cell to express a foreign protein.

## [IV] Transformant

The transformant is obtained by transforming an animal cell with the plasmid as described in [II] or by cotransforming an animal cell with two plasmids③ and④ as described in [III].

Examples of animal cells which may be used in the present invention and which are useful as a cell line include VERO cell, HeLa cell, Chinese hamster ovary (CHO) cell line, WI38 cell, BHK cell, COS-7 cell, MDCK cell line, CI27 cell, HKG cell and human kidney cell line. More specific examples are Chinese hamster ovary cell (CHL-KI: ATCC CCL61), newborn hamster kidney cell (BHK: ATCC CCLI0), SV-40 cell (COS-7, CV-I: ATCC CRLI651) and African green monkey kidney cell (Vero: ATCC CCL-8I).

As such cells, it is particularly preferred that they are DHFR-deficient cells.

The transformation of an animal cell may be performed by known methods, for example, calcium phosphate precipitation, protoplast polyethylene glycol fusion and electroporation.

For amplification with MTX (methotrexate), a transformat is cultured in medium containing 10 nM to 4 nM MTX. MTX may be stepwise increased or may be kept in a high concentration from the initial stage and performed in a single step, and a resistant strain may be selected in the medium.

As the medium, there may be used MEM-alpha, I-10% FCS and D-MEM. Conditions for culturing are between 10 and 37°C for approximately I to 200 hours.

## [IV] Process for producing foreign protein

The transformant as described in [III] above is cultured to express the foreign protein in a conventional manner.

In the present invention, a DNA encoding DHFR is arranged under the control of UK promoter to amplify a foreign protein expression. It is noted from the arrangement that the degree of amplification is much superior to the known level.

Accordingly, the present invention is considered to be extremely useful for the mass production of a foreign protein using an animal cell as a host in the field of genetic engineering.

The present invention is explained in more detail with reference to the following examples but is not deemed to be limited thereto.

Example I

[I] Construction of pHR24

Plasmid in which DNA sequence encoding human prourokinase (PUK) having asparagine-bound sugar chain at EGF domain was constructed as described below.

An expression vector of modified human PUK having a new sugar chain binding site at the 24-position within the EGF domain of PUK wherein Tyr24 of natural human PUK is replaced with Asn24 was prepared. Preparation of the expression vector is described below in detail. In this example, synthetic DNA was purified by modified polyacrylamide gel. Enzymes such as restriction enzymes and T4 DNA polymerase, kits such as M I3 sequencing kit and ligation kit, and E. coli HBI0I and JMI09 competent cells manufactured by Takara Shuzo Co., Ltd. were used. Calf testine alkaline phosphatase manufactured by Boehringer Mannheim Co., Ltd. were used. Recombinant DNA technique was performed according to a modified method of the method described in "Molecular Cloning", Cold Spring Harbor, New York: Cold Spring Harbor Laboratory (I982).

(i) Synthesis of oligonucleotide

Using automated DNA synthesizer (38IA, manufactured by Applied Biosystem Co., Ltd.), 2 single stranded DNAs (DNA-I and DNA-2) were prepared. The respective nucleotide sequences are shown below. Tyr24 is replaced with Asn24 to which sugar chain is attached.

EP 0 457 527 A1

```
                      KpnI                          SspI
5'-CGAACTGTGACTGTCTAAATGGAGGTACCTGTGTGTCCAACAAGAACTTCTCCAATATTCAC
3'-TTGACACTGACAGATTTACCTCCATGGACACACAGGTTGTTCTTGAAGAGGTTATAAGTG

  AsnCysAspCysLeuAsnGlyGlyThrCysValSerAsnLysAsnPheSerAsnIleHis


    TGGTGCAACTGCCCAAAGAAATTCGGAGGGCAGCACTGTGAAAT-3'     DNA-1

    ACCACGTTGACGGGTTTCTTTAAGCCTCCCGTCGTGACACTTTAGC-5'   DNA-2

    TrpCysAsnCysProLysLysPheGlyGlyGlnHisCysGluIle
```

DNA-I and DNA-2 contains DNA sequence in which 24-tyrosine residue of PUK [M. Nagai et al., Gene., 36, I83-I88 (I985)] is replaced with asparagine residue. Furthermore, GGAACA was converted into GGTACC to introduce Kpn I site therein. AACATT was converted into AATATT to introduce Ssp I site therein.

After splitting off the protective group, 64/I00 of the synthesized oligonucleotide based on the total amount synthesized was purified by I2% acrylamide urea gel electrophoresis to give I9.0 μg of DNA-I and 2I.I μg of DNA-2 as single stranded DNAs.

(ii) Construction of plasmid

Outlined construction of pTT03 and pHR24 is shown in Fig. I.

Construction of pTT03

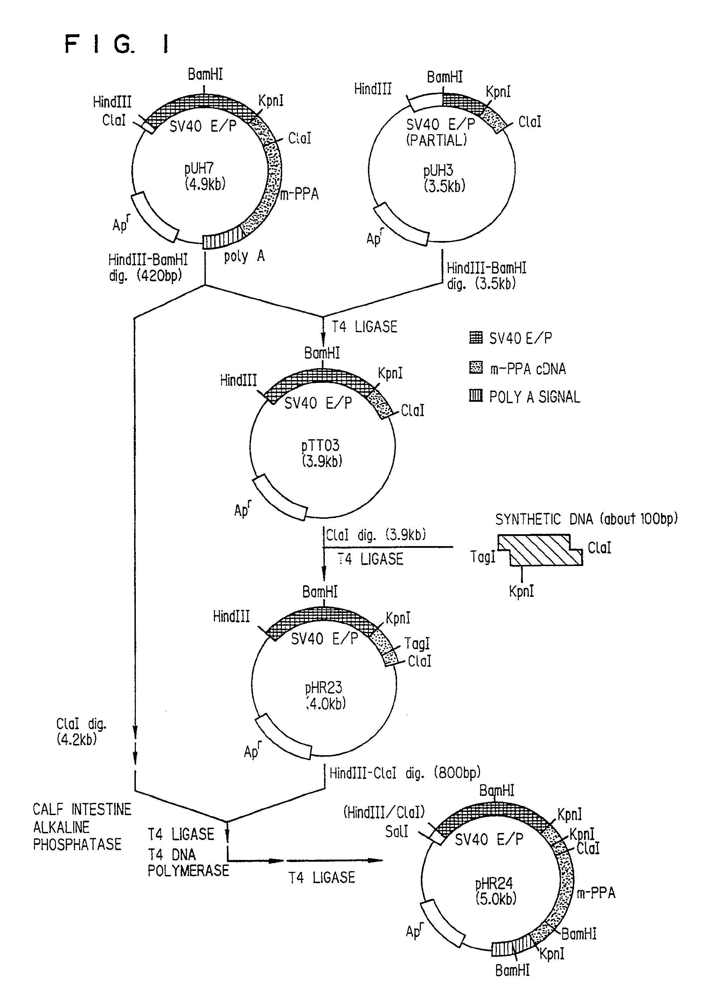

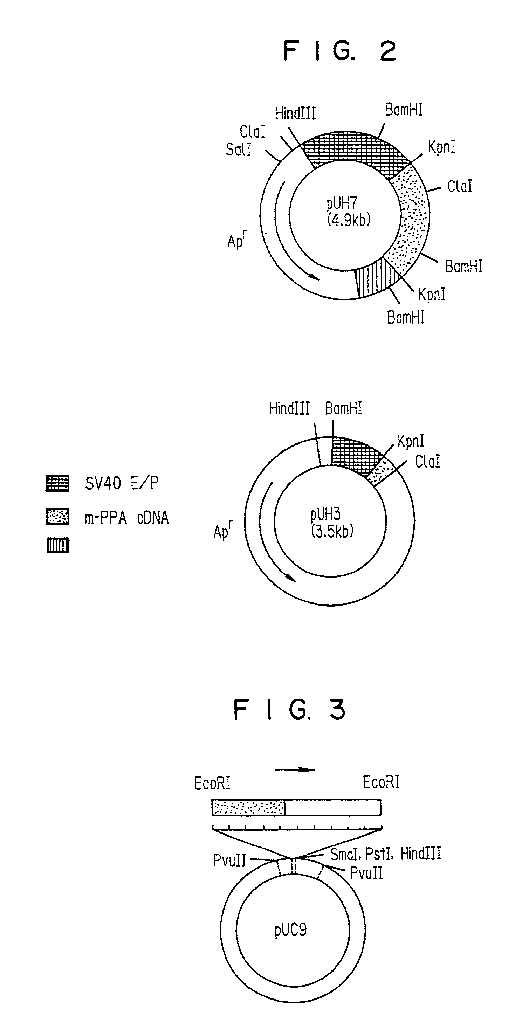

Plasmid pTT03 containing enhancer/promoter domain derived from SV40 and signal sequence of plasminogen proactivator (PUK) as well as gene encoding N-terminal 4 amino acids of PUK was constructed. In order to obtain 5' region of SV40 enhancer/promoter at the BamH I site, pUH7 (Japanese Patent Application Laid-Open No. 63-I46789) was digested with Hind III and BamH I, and 420 bp fragment was isolated and purified by agarose gel electrophoresis. On the other hand, pUH3 (Japanese Patent Application Laid-Open No. 63-I46789) was also digested with Hind III and BamH I. Linear 3.5 kb fragment was isolated and purified by agarose gel electrophoresis. This fragment was ligated with the 420 bp fragment using DNA ligase. A plasmid which gave 3.5 kb and 420 bp fragments after digesting with Hind III and BamH I was named pTT03. Twelve transformats were confirmed but all were transformats carrying pTT03 (cf. Fig. 2).

Construction of pHR23

pHR23 containing enhancer/promoter of SV40, signal sequence of PUK and Tyr24 of PUK replaced by asparagine residue was constructed.

After pTT03 was digested with Cla I, the resulting linear 3.9 kb fragment was isolated and purified by agarose gel electrophoresis. The fragment was subjected to terminal dephosphorylation with calf testine alkaline phosphatase. On the other hand, after the kination of the oligonucleotides DNA-I and DNA-2 synthesized in (i) described above, annealing was performed followed by ligation with the dephosphorylated 3.9 kb fragment. A plasmid which gave 800 bp DNA fragment after digestion with Hind III and ClaI was named pHR23. As the result of screening of I2 transformats, 8 out of I2 were transformats bearing the desired plasmid. The portion having the synthetic DNA in plasmid was also confirmed by determining its nucleotide sequence.

Construction of pHR24

pHR23 has DNA sequence encoding the region from N-terminal residue to 44-isoleucine residue of PUK. Therefore, DNA encoding the region from 45-aspartic residue to C-terminal residue was ligated with pHR23 to construct pHR24.

pUH7 was digested with Cla I and the resulting 4.2 kb fragment was isolated and purified by agarose gel electrophoresis. The fragment was subjected to terminal dephosphorylation with calf testine alkaline phosphatase. The dephosphorylated product was ligated with a 800 bp fragment obtainined by digesting pHR23 with Hind III-Cla I, utilizing Cla I site. The Hind III site and another Cla I site were rendered blunt end with T4 DNA polymerase followed by ligation again. In primary screening of 24 transformations, a size of circular plasmid was used as an index. That is, comparison was made with circular plasmid of pUH7 which was smaller by I00 bp than the desired plasmid. As the result, 5 out of 24 transformats were plasmids having the desired size. By this procedure for construction, there was a possibility that the Hind III/Cla I fragment inserted therein might be ligated in the inverted direction. Therefore, screening of the plasmid which gave 900 bp fragment by Kpn I digestion was then performed. The results reveal that one out of 5 transformats was the desired plasmid.

[II] Construction of pTT06

plasmid having a DNA encoding dihydrofolate reductase (DHFR) ligated with a promoter (SV40) capable of regulating the expression of a foreign protein in an animal cell and having an urokinase (UK) promoter was constructed as described below.

(i) Construction of pUKP-I: Preparation of DNA encoding urokinase (UK) promoter

(l) Preparation of probe

Plasmid pUK4 (Japanese Patent Application Laid-Open No. 6l-I77987) containing a part of human urokinase cDNA was digested with Pst I. After electrophoresis on I% agarose gel, about 400 bp fragment was recovered by electroelution. By multiple priming as described below, 0.4 μg of this DNA fragment was labeled with [α-$^{32}$P]dCTP (Amersham, PBI0205).

```
DNA fragment: 0.4 μg (8 μl)

    │    ◄── after heating at 100°C for 3 minutes,
    │        quenching on ice water;

    │    ◄── 15 μl of multiple priming solution was
    │        added;

    │    ◄── 25 μl of [α-³²P]dCTP (250 μCi) was
    │        added;

    │    ◄── incubation at room temperature for 1.5
    │        hours;

    │    ◄── The reaction solution was applied to a
    │        nick column which had been equilibrated
    │        with column buffer (0.1% SDS-1 mM EDTA-10
    │        mM Tris-HCl, pH 7.4);

    │    ◄── fractionation by 100 μl each
    │
```

The desired fraction was collected and Cerenkov count was examined. As the result, the count was 9.69 x I0$^7$ cpm. Yield of the label and specific radiation activity of the label were determined to be 4I% and 2.4 x I0$^8$ cpm/μg, respectively.

(2) Southern hydridization of HKG cell DNA

According to Riccio et al. Nucl. Acids. Res., 13, 2759-277I (I985), human urokinase promoter domain is obtained as 5.8 kb Eco RI fragment and I2 kb BamH I fragment of human chromosomal DNA. After the high molecular DNA of HKG cell was digested with Eco RI and BamH I, respectively, thus obtained I0 μg each was subjected to electrophoresis on 0.8% agarose gel followed by confirmation with Southern hydridization using the probe prepared in (I). As the result, signals were detected at the positions corresponding to the respective sizes.

(3) Preparation of 5.8 kb Eco RI fragment

The high molecular DNA (200 μg) of HKG cell was digested with I000 units of Eco RI at 37°C overnight. After electrophoresis on 0.8% agarose gel (Takara Shuzo Co., Ltd., HE-I2 apparatus for electrophoresis), staining with ethyldium bromide was performed to use as a marker for electrophoresis analysis. Centering around the 5.8 mb position determined from the product by digesting γ-DNA with Hind III, gel was cut out in a width of 2 mm (Gel 2) and further gel above the 5.8 kb position (Gel I) and gel below the 5.8 position (Gel 3) were cut out in a width of 3 mm, respectively. The DNA fragments were extracted from those gels by electroelution. A part of the individual extracted DNA was subjected to electrophoresis on 0.8% agarose gel. It was confirmed by Southern hybridization whether the desired DNA fragment was contained. It was presumed that the desired urokinase promoter domain would be present in the DNA fragment extracted from Gel 2.

## (4) Production of DNA library and screening

With respect to the DNA fragment extracted in the preceding, DNA library was prepared using phage vector $\gamma_{gt}$10. $6.5 \times 10^5$ recombinant phages were subjected to primary screening by plaque hybridization. By the primary screening, 28 positive clones were obtained. The clones were further subjected to secondary screening by plaque hybridization, whereby 5 positive clones were selected. Total DNA was extracted from the positive recombinant phage clones by a simple extraction method. After digestion with Eco RI, electrophoresis was performed on I% agarose gel followed by Southern hybridization. As the result, it was judged that 2 recombinant phages were positive. Clones I, 4 and 5 were derived from the same clone and thus regarded as the same. Clone I5 was derived from different clone.

## (5) Subcloning of 5.8 kb Eco RI fragment

Phage DNA was prepared by a simple extraction method, from the clones determined to be positive by Southern hybridization. The phage DNA was digested with Eco RI. The digestion product was extracted with phenol-chloroform. The aqueous phase was further extracted with chloroform followed by ethanol precipitation. After digesting plasmid pUC9 (Pharmacia Fine Chemicals, I.c.) with Eco RI, the digestion product was treated with alkaline phosphatase. A part (I μg) of the product was ligated with the product by digesting the phage DNA with Eco RI to transform E. coli HBI0I. Plasmid DNA was extracted from some of the transformats by a simple extraction method. After digestion with Eco RI, the digestion product was subjected to I% agarose gel electrophoresis. As the result, some subclones had the DNA fragment corresponding to 5.8 kb. After digesting these Clones I and 4 with various restriction enzymes, the digestion products were subjected to electrophoresis on I% agarose gel. In clones I and 4, some fragments in common were found, though cleavage pattern was different. It was thus presumed that the 5.8 kb fragment would be inserted in the inverted direction. The plasmids were named pUKPI (cf. Fig. 2) and pUKP2, respectively.

## (6) Treatment of p-UKPI with restriction enzyme

Based on restriction enzyme map deduced from the nucleotide sequence of the human urokinase gene reported by Riccio et al. (supra), it was confirmed that the enzyme fragment in the restriction enzyme map would be obtained. E. coli HBI0I bearing pUKPI was cultured at 37°C overnight in I00 ml of super-broth supplemented with 40 μg/ml ampicillin. Then plasmid DNA was prepared by an alkali-SDS method. The DNA was treated with various restriction enzymes. Sizes of the presumed restriction enzyme fragments of the plasmid and their presence or absence are shown in Table I. As shown in Table I, the presumed fragments were all detected and it was thus confirmed that the DNA fragment encoding the desired human urokinase promoter site was cloned.

### Table 1
### Presence or absence of restriction enzyme fragments

| | pUKP1 | |
|---|---|---|
| Enzyme | Size of Fragment (bp) | Presence of Absence |
| Bgl II | 270 | ◯ |
| Sma I | 2260 | ◯ |
| Pvu II | 695 | ◯ |
| | 1640 | ◯ |
| | 792 | ◯ |
| Pst I | 682 | ◯ |
| | 946 | ◯ |
| | 584 | ◯ |
| Hind III | 1816 | ◯ |
| Sma I + ECoRV | 590 | ◯ |
| EcoRI + EcoRV | 3050 | ◯ |
| Hind III + EcoRV | 1283 | ◯ |
| BamH I + Bgl II | 595 | ◯ |
| Bgl II + EcoRV | 2205 | ◯ |

◯ : DNA fragment of the size was detected.

(7) Confirmation of nuceotide sequence in part of pUKPl

With respect to the plasmid DNA prepared above, a part of the nucleotide sequence was examined by a dideoxy method. The results coincided with the report of Riccio et al. (supra).

(ii) Construction of pTT06

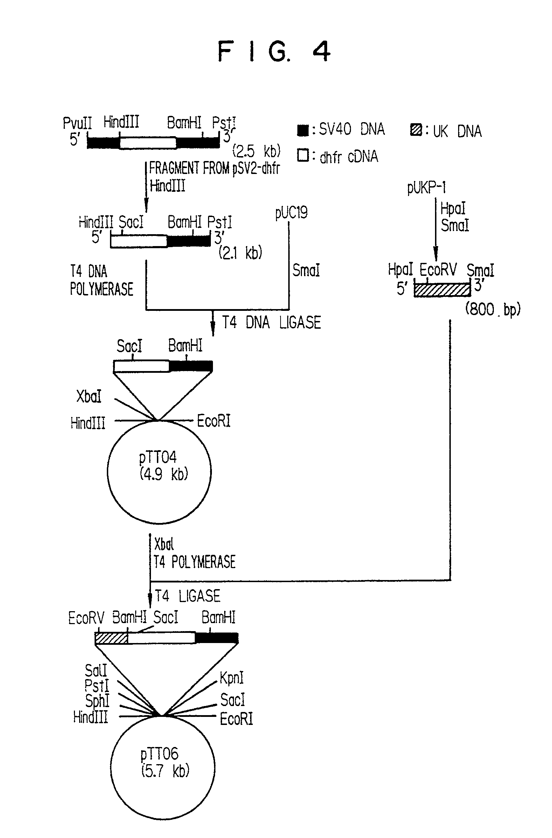

Plasmid containing UK promoter, dhfr cDNA and SV40 poly-A was constructed as described below. Outlined construction of plasmid is shown in Fig. 4.

2.5 kb DNA fragment containing SV40 enhancer/promoter obtained by treating pSV2-dhfr (Japanese Patent Application Laid-Open No. 63-l05675) with Pvu II and Pst I, dhfr cDNA and SV40 late poly-A additional signal was further digested with Hind III to give SV40 enhancer/promoter-free 2.l kb DNA fragment. After this Hind III/Pst I DNA fragment was rendered blunt end with T4 DNA polymerase, the DNA fragment was cloned on pUCl9 (Takara Shuzo Co., Ltd.) at the Sma I site. As the result, pTT04 in which 5′ end (N-terminal of protein) of dhfr cDNA was directed to the Hind III side of pUCl9 polylinker was obtained. The plasmid was confirmed by the digestion with BamH I, in terms of size of the DNA fragment cut out with BamH I in pUCl9 polylinker and with BamH I downstream of the SV40 poly-A additional signal. The I.6 kb DNA fragment was obtained from pTT04. Next, pUKP-I was digested with Hpa I and Sma I to recover about 800 bp DNA fragment excised at the Sma I site about 30 bp downstream from the urokinase gena transcription initiation site and at the Hpa I site about 800 bp upstream from the initiation site. The DNA fragment containing the UK promoter site was inserted upstream of dhfr cDNA of pTT04. That is, pTT04 was digested with Xba I and then treated with BAP followed by rendering blunt end with T4 DNA polymerase. Thereafter, the ligation with Hpa I/Sma I fragment of UK promoter was performed. After transformation, clones in which dhfr gene and UK promoter were arranged in the same transcription direction were selected from the resulting colonies. Plasmid which gave 280 bp DNA fragment by digestion with Eco RV at 5′ end of the UK promoter DNA fragment and by digestion at pOCI9 with Sal I was selected from pTT04 and named pTT06. The plasmid was further digested with Eco RV and Sac I, and Eco RV and BamH I, respectively, to confirm its structure. Digestion of pTT06 with Eco RV and Sac I gave 0.9, I.8 and 2.9 bp fragments and, digestion with Eco RV and BamH I gave 0.6, I.6 and 3.5 kb fragments. Sizes of these fragments coincided with the restriction enzyme map of the desired plasmid.

## [III] Preparation of pTT08 and pTT09

Plasmid containing DHFR transcription unit under the control of UK promoter and Asn$^{24}$-PPA transcription unit under the control of SV40 promoter was constructed as described below.

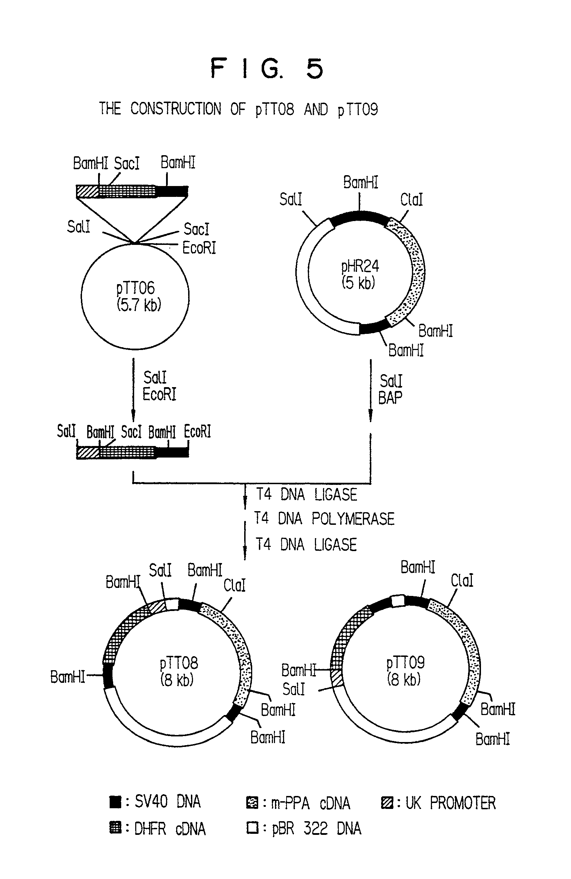

Outlined construction of plasmid is shown in Fig. 5.

pTT06 was digested with Sal I and Eco RI to give 2.9 kb fragment containing UK promoter, DHFR cDNA and SV40 late poly-A additional signal. On the other hand, pHR24 prepared in [I] above was digested with Sal I to render linear strand. The digestion product was then subjected to 5′ dephosphorylation with BAP. These two DNA fragments were ligated with each other to ligate one Sal I site of pHR24 with the Sal I site of UK-DHFR DNA fragment. Subsequently, T4 DNA polymerase was acted on the ligation product to render the remaining Sal I site of pHR24 and the Eco RI site of UK-DHFR DNA fragment blunt end, respectively, and then ligation was again performed. Using the thus obtained ligation solution, E. coli HBI0I was transformed. pTT08 in which DHFR transcription unit and m-PPA transcription unit were arranged in the inverted direction and pTT09 in which these units were arranged in the same direction were obtained from the transformats. The plasmids were confirmed by double digestion with Cla I and Sal I and by digestion with BamH I, respectively. pTT08 gave 7.2 kb and 0.8 kb fragments by digestion with Cla I and Bal I and 3.I, I.6, I.4, I.3 and 0.3 kb fragments by digestion with BamH I. On the other hand, pTT09 gave 4.2 kb and 3.8 kb fragments by digestion with Cla I and Sal I and 3.4, I.6, I.4, I.0 and 0.3 kb fragments by digestion with BamH I.

## [III] Introduction into animal cell

### (i) Material and method

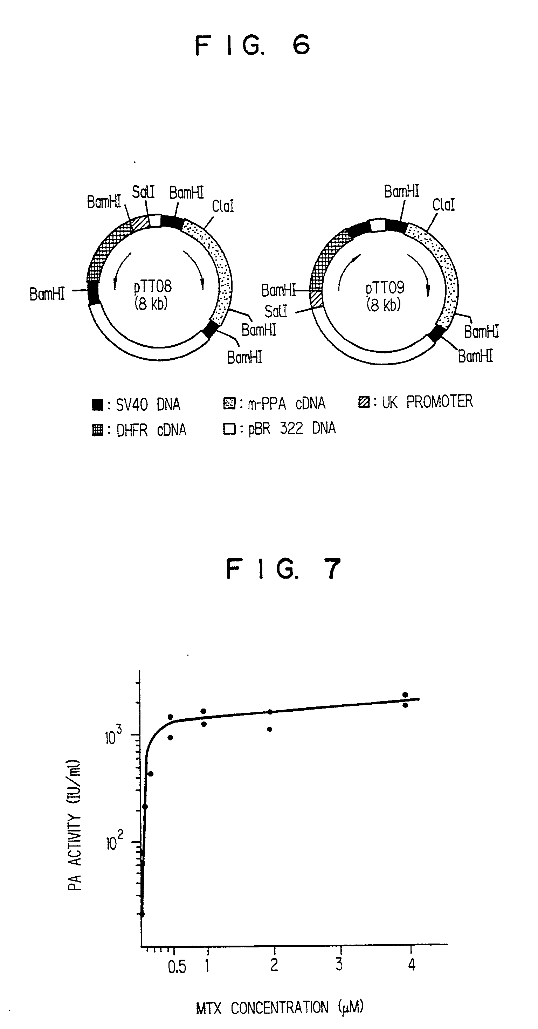

#### (I) Plasmid DNA (cf. Fig. 6)

pTT08: Plasmid in which DHFR transcription unit under the control of UK promoter and Asn$^{24}$-PPA transcription unit have been arranged in the inverted direction.

pTT09: Plasmid in which Asn$^{24}$-PPA transcription unit under the control of SV40 promoter has been arranged downstream of DHFR transcription unit under the control of UK promoter in the same direction.

#### (2) Cell

CHO-KI cell-derived DHFR-deficient strain.

The strain was prepared and amplified according to the method described in Proc. Natl. Acad. Sci. (USA), 77, 42I6 (I980).

#### (3) Methotrexate (MTX)

(+) Amethopterin manufactured by Sigma Co. was dissolved in 0.I4 M NaCl and 0.02 M HEPSE (Nakarai Tesque) to prepare 2 mM stock solution. The stock solution was added to medium in a desired concentration, which was provided for use.

(4) Medium and serum

MEM-α (containing ribonucleoside and deoxyribonucleoside) (Gibco).
MEM-α (containing neither ribonucleoside nor deoxyribonucleoside) (Gibco).
These media are abbreviated as MEM-α (w) and MEM-α (w/o), respectively.
As serum, fetal calf serum (Mitsubishi Chemical Industry Co., Ltd., MHOl) was used after immobilization.

(5) Transfection of DNA and selection of transfectant

DXB-ll cells subcultured in MEM-α (w) supplemented with l0% FCS were treated with trypsin (0.25%, trypsin, 0.02% EDTA) to peel apart from a dish and suspended in Hanks' solution in l0[7] cells/ml. By electroporation, 5 μg of plasmid DNA was transfected to 0.5 ml of the suspension (5 x l0[6] cells). The cells were inoculated on 5 dishes of l0 cm each in l0[6] cells/dish. After culturing in MEM-α (w) containing l0% FCS for 2 days, the medium was exchanged with MEM-α (w/o) containing l0% FCS which was a selection medium. The medium was exchanged every 2 or 3 days. After culturing for about l0 days, colonies were formed. Each colony was transferred to a 96 wells plate followed by further incubation. At the time when the cells became almost dense in the 96 wells plate, plasminogen activator (PA) activity in the various culture supernatants was determined. The cell which gave the highest activity was scaled up and cloned again. The clone was subjected to amplification with MTX.

(6) Amplification of the transfected gene with MTX

The Asn[24]-PPA-producing cells (2 ml) obtained in (5) above were inoculated on MEM-α (w/o) containing l0% FCS and l0 nM MTX charged in a 6 cm dish (Falcon, 3802) in a concentration of 5 x l0[4] cells/ml. Considerable cells were dead in 3 to 4 days incubation. However. when exchange of the medium was continued for every 3 other days, growth of the cells was noted. When the cell count became sufficient in 2 to 4 weeks, the cells were subcultured in medium having a MTX concentration of next stage. As such, MTX concentration was gradually increased by 2 to 4 times each, starting from l0 nM of MTX concentration. MTX-resistant cells in each concentration were cultured in a 6 cm dish or a l0 cm dish (Falcon, 3803) and the PA activity in each supernatant was determined.

(7) Determination of PA activity in the culture supernatant

The PA activity in the culture supernatant of each cell was determined by a fibrin plate method [Astrup, T. and Mullertz, S. Arch. Biochem. Biophys., 40, 346-35l (l952)] and a peptide MCA method [T. Morita et al., J. Biochem., 82, l495 (l977)].
An amount of the single stranded pro enzyme in a sample was determined as follows. That is, each activity of a plasmin-treated sample and aplasmin-non-treated sample was determined by the peptide MCA method. A value obtained by subtracting the activity of the plasmin-non-treated sample from the activity of the plasmin-treated sample was defined as the activity of single stranded pro enzyme.
As the standard for the determination of the activity, urokinase comparison standard (manufactured by The Green Cross Corporation, Lot. S-004) was used.

(ii) Results

(l) Production in early transfectant Asn[24]-PPA

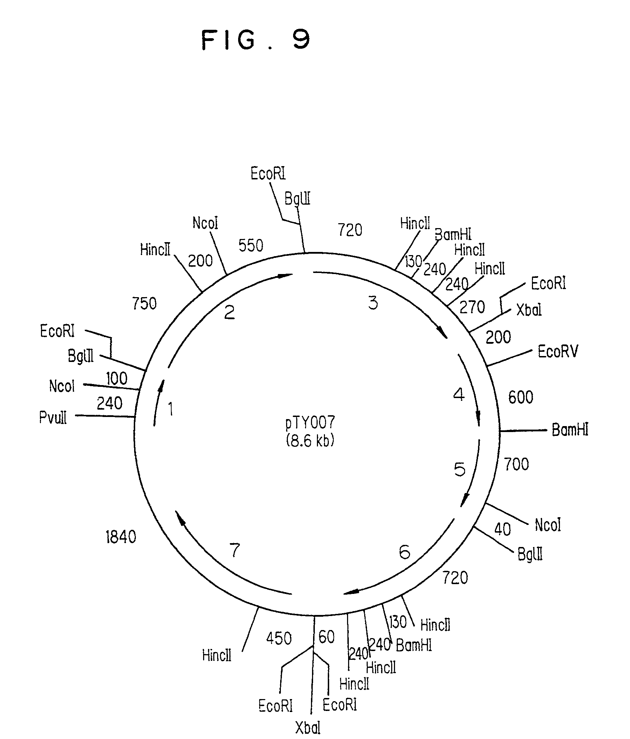

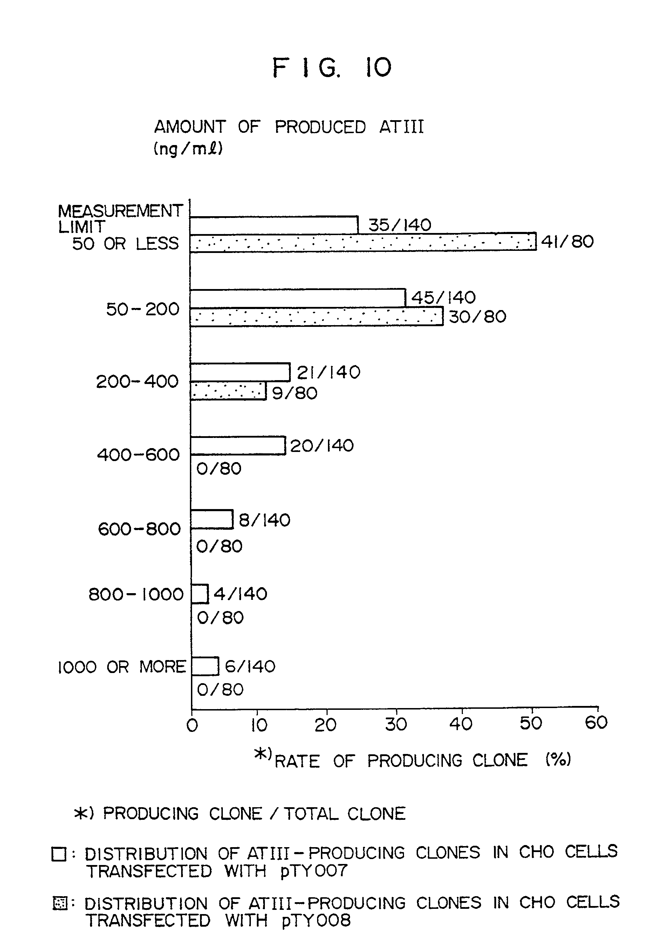

Plasmids pTT08 and pTT09 which are different from each other in the positional relationship between dhfr gene and Asn[24]-PPA as shown in Fig. 9 were transfected to DHFR-DHFR-deficient CHO cells. The cells lack DHFR activity and cannot synthesize nucleotide. In order to select the cells transfected with DNA encoding dhfr, transfectants were selected in nuceoside-free MEM-α (w/o) medium. The colonies formed in the medium were transferred to a 96 wells plate. When the cells became almost dense, the PA activity in the supernatant was determined by the fibrin plate method. The results are shown in Table 2.
Among the colonies obtained, almost the half showed the PA activity. The expression of PA was not affected by the difference of the plasmid.
Among these cells, the transfectants showing the activity of more than l0 IU/ml were scaled up and the activity was again determined. The cells (cells obtained by transfecting pTT08 to DHFR-deficient CHO cells) showing 47 IU/ml in a l0 cm dish were provided for amplification test with MTX. Even though the cells were again cloned, the resulting clones all showed the PA activity of 20 IU/ml in a 96 wells plate, indicating that the cells were originally a homogeneous cell mass.

Table 2  Amount of m-PPA produced from DHFR-deficient CHO cell transformed by plasmid constructed from DHFR and Asn$^{24}$-PPA transcription unit

Count of Colony (IU/ml)*

| Plasmid | Total | 0 | 0-3 | 3-6 | 6-12.5 | 12.5-25 | 25-30 |
|---------|-------|----|-----|-----|--------|---------|-------|
| pTT08 | 23 | 12 | 3 | 0 | 5 | 3 | 0 |
| pTT09 | 31 | 16 | 6 | 1 | 6 | 0 | 2 |

* Each cell was cultured in 96 wells and the PA activity was determined by the fibrin plate method.

(2) Increase in amount of Asn$^{24}$-PPA produced by the addition of MTX

By adding MTX to the cells selected in (I), it was examined if DNA encoding dhfr could be amplified and at the same time, DNA encoding Asn$^{24}$-PPA proximal thereto could also be amplified thereby to increase an amount of Asn$^{24}$-PPA produced. Relationship between concentration of MTX and PA activity of the supernatant in each resistant cell is shown in Fig. 7. The concentration of MTX started from I0 nM but almost all cells were dead in 3 to 4 days. In the transfectants of plasmid bearing the transcription unit of dhfr-encoding DNA with SV40 promoter, the cells were not injured very seriously even though the concentration of MTX started from 50 nM. The amount of Asn$^{24}$-PPA produced was not remarkably increased with I0 nM MTX-resistant cells, as compared with early transfectant. However, as the MTX concentration increased to 50, I00, 200 and 500 nM, the production amount increased almost proportionally. The production amount showed 20 to 50 IU/ml with early transfectant by culture for 4 days in an incubation cell count of 200 x I0$^4$ cells/I0 cm dish. However, the production amount became approximately I000 IU/ml (6.7 mg/l) with 500 nM MTX-resistant cells.

The MTX concentration was further increased to I μM, 2 μM and 4 μM to examine the PA activity in the supernatant of each resistant cell. However, only some increase was noted and the maximum was I000 to 2000 IU/ml under the culture conditions.

(3) Production of Asn$^{24}$-PPA in high concentration MTX-resistant cell

With respect to cells resistant to MTX showing 0.5 μM or more, profile of Asn$^{24}$-PPA production was examined in both medium with MTX and medium without MTX.

The cells were inoculated on a I0 cm dish in 200 x I0$^4$ cells/I0 ml and the PA activity of the supernatant was determined by the fibrin plate method 2 days after the incubation. The cells formed almost full sheet in 3 to 4 days under the conditions and were subject to injury on or after day 5 or 6 so that the cells began to be peeled apart. The cells cultured in medium with MTX showed somewhat slowed cell growth that with the cells in medium without MTX. The PA activity in the supernatant was higher in the medium without MTX, probably because the cell count was reflected. All cells showed the highest PA activity 4 or 5 days after the incubation and no substantial increase in the activity thereafter. Under the conditions, the PA activity reached I800 to 2000 IU/ml (I2 to I3 mg/l) with the cells resistant to I μM MTX and 2700 to 3200 IU/ml (I8 to 2I mg/l) with the cells resistant to 2 μm and 4 μM MTX. However, with the cells resistant 8 μM MTX, the production amount was somewhat decreased, indicating the PA activity of only 2000 IU/ml. The progress of 4 μM MTX-resistant cells in Asn$^{24}$-PPA production is shown in Table 3.

When the cells formed a full sheet, the cell count was approximately I0$^7$ cells/dish. Thus, the maximum daily production amount was 200 IU (I.3 μg)/ml/I0$^6$ cells/day.

13

(4) Content of single strand in Asn[24]-PPA produced

The PA activity in the culture supernatant was determined by the peptide MCA method to examine an amount of single stranded pro enzyme in a sample. The results reveal that the single strand content in each sample was about 85% after culturing for 3 to 4 days. One day culture maintained the single strand content of 90%.

Table 3. Production amount of Asn[24]-PPA in 4 μM MTX-resistant cell [a)]

| Number of days cultured | PA activity given by fibrin plate method | | | | | |
|---|---|---|---|---|---|---|
| | Medium with MTX | | | Medium without MTX | | |
| | First | Second | Third | First | Second | Third |
| 2 | 707 | 1020 | 839 | 657 | 1033 | 839 |
| 3 | 1101 | 1355 | 1038 | 1572 | 1476 | 1332 |
| 4 | 1744 | 2183 | 1965 | 2185 | 2498 | 1869 |
| 5 | 2317 | 2228 | 2075 | 3037 | 2360 | 2295 |
| 6 | 2969 | 2312 | 2110 | 2983 | 2379 | 2049 |
| 7 | 2800 | 2066 | | 2890 | 2335 | |
| 8 | 2328 | | | 2738 | | |

a) The cells were inoculated on MEM-α with MTX or without (w/o) MTX, supplemented with 10% FCS, in 200 x $10^4$ cells/10 ml/10 cm dish.

Comparative Example

[IV] Comparison in production amount with the prior art

Using pSV 2-dhfr (ATCC 37I46: plasmid in which DNA encoding DHFR is arranged downstream of SV40 promoter) and pHR24, a plasmid in which DHFR transcription unit under the control of SV40 promoter and Asn[24]-PPA transcription unit under the control of SV40 promoter were arranged in the inverted direction was constructed in a conventional manner. The plasmid was used as comparison. Using DHFR-deficient CHO cells which acquired resistance to 2 μm MTX by the signal step, the production amount of Asn[24]-PPA was compared between the comparative plasmid (SV40 promoter type) and the plasmid of the present invention (UK promoter type). The results are shown in Table 4.

Table 4

| | Promoter for expressing DHFR | Production amount of $Asn^{24}$-PPA ($IU/10^6$ cells) |
|---|---|---|
| Comparative plasmid | SV40 promoter | 51 |
| Plasmid of this invention | UK promoter | 3000 |

Example 2

The procedures were carried out in a manner similar to Example I [IV] except that pHR24 (I0 μg) prepared in Example I [I] and pTT06 (I μg) prepared in Example I [II] were simultaneously transfected to 5 x I0⁶ of DHFR-deficient strain derived from CHO-KI cells, and then the expression of $Asn^{24}$-PPA was confirmed in the same way as in Example I.

Example 3

[I] Construction of pTY007 and pTY008

Plasmid containing DHFR transcription unit under the control of UK promoter and human ATIII transcription unit under the control of SV40 promoter was constructed as described below.

(i) Synthesis of oligonucleotide

Using automated DNA synthesizer (supra), 2 kinds of probes (Probe-I and Probe-2) were prepared. The respective nucleotide sequences are shown below.

Probe-I (DNA complement to 5I nucelotide sequences from translation initiation codon):

5'-ATAAACCTTCCTTTTTCCAGAGGTTACAGT

TCCTATCACATTGGAATACAT-3'

Probe-2 (DNA complement to 5I nucleotide sequences from glycine residue at 244-position of matured ATIII):

5'-CAGGCTCTTCTCAGGCTTGGGCAAGATGAG

gaccatggtgatgtcatcacc-3'

Primer-I (DNA of 32 nuceotides containing translation termination codon (underlined); 4 nuceotides (*) are sub-stituted):

          **     **

5'-GTGTTAAGTAAAAGCTTCGAATTCTTTGCA

CC-3'

15

(ii) Cloning of cDNA encoding human ATIII

Messenger RNA (mRNA) of human hepatic cells was isolated using oligo dT column (Chirgwin et al., Biochemistry, 18: 5294-5299, 1979) and subjected to size fractionation by sucrose density gradient centrifugation (Schweinfest et al., Proc. Natl. Acad. Sci, USA, 79: 4979, 1982). Using γgtl0 (Huynth et al., DNA Cloning, l: 49, 1985) as vector, cDNA was prepared from mRNA of an appropriate size (up to 28S) in a conventional manner (Okayama et al., Mol. Cell. Biol., 2: 161, 1982). Using the two probes (supra) prepared based on the nucleotide sequence of ATIII cDNA previously reported, plaque hydridization was performed (Benton et al., Science, 196: 181, 1977). Positive clones were isolated and phage DNA was prepared. By digestion with Eco RI, about 1500 bp cDNA insert was excised and recloned on plasmid pUCII8 (Vieira et al., Meth. in Enzymology, 153: 3, 1987). This plasmid was named pEA001 and its nuceotide sequence was identified by the dideoxy method (Sanger et al., Proc. Natl. Acad. Sci. USA, 74: 5463, 1977).

(iii) Preparation of plasmids (pTY007, pTY008) for the expression of ATIII

The plasmids are prepared using the following 3 plasmids.

pEA003:

A plasmid obtained by incorporating ATIII cDNA into pUCII8, in which Eco RI site and Hind III site are introduced upstream of the poly-A additional signal of ATIII cDNA according to site-directed mutagenesis.

pSVII-dhfr:

pSVII-dhfr is a plasmid obtained by introducing Xba I site into pSV2-DHFR around its Eco RI site, which contains SV40-derived early promoter and enhancer, mouse dhfr cDNA, SV40-derived splicing junction and poly A additional signal.

pTT06:

This plasmid corresponds to pTT06 of Example I.

## Construction of pEA003

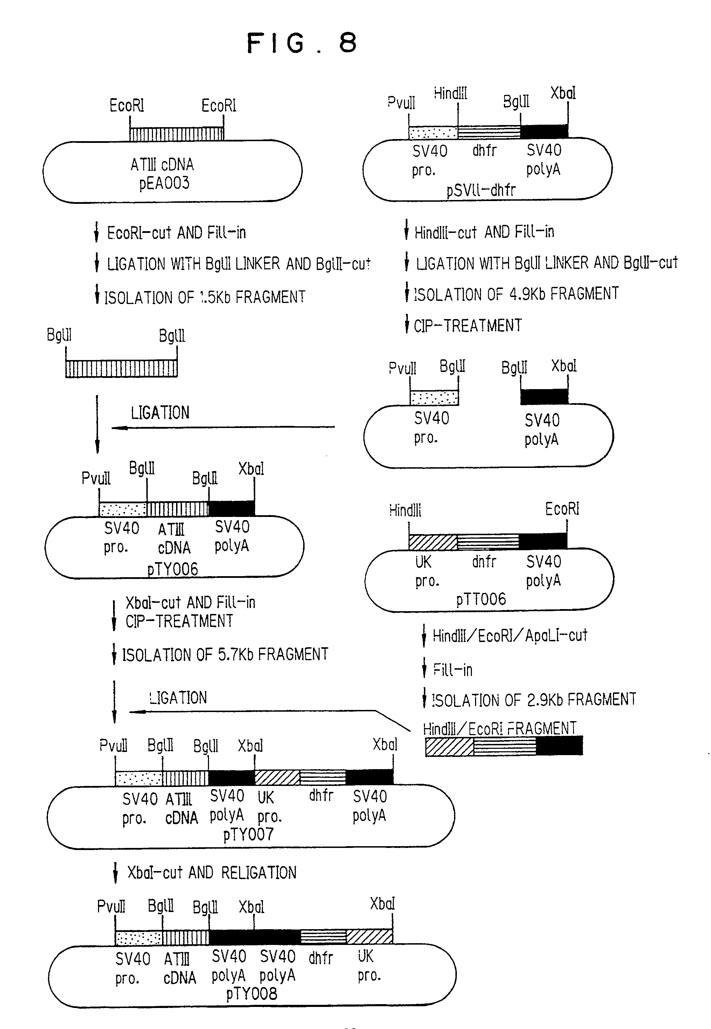

According to the site-specific nucleotide replacement method (Sayers et al., Nucl. Acids Res., 16: 791, 1988, Hind III and Eco RI sites were provided between the region coding for ATIII in pEA001 and polyadenylic acid tail, using the primer (supra). This plasmid was named pEA003 (cf. Fig. 8). By digesting with Eco RI, polyadenylic acid tail-free about 1400 bp ATIII cDNA can be isolated from pEA003.

## Construction of pSVII-dhfr

pSV2-dhfr (supra) was digested with Eco RI. After rendering blunt end with DNA polymerase I (Klenow fragment), Xba I linker was inserted using T4 DNA ligase. This plasmid was named pSVII-dhfr.

## Construction of pTT06

The procedures were performed as described in Example I.

## Construction of pTY007 and pTY008

Construction of plasmids for expressing ATIII (pTY007 and pTY008; is shown in Fig. 8. Firstly, ATIII cDNA was excised from pEA003 using Eco RI. After fill-in, ATIII cDNA was ligated with Bgl II linker. On the other hand, pSVII-DHFR was digested with Hind III. After fill-in, the digestion product was ligated with Bgl II linker. By further digesting with Bgl II, mouse dhfr cDNA was removed. These products were ligated to construct plasmid (pTY006) capable of expressing ATIII cDNA under the control of SV40 promoter.

On the other hand, pTT06 was digested with restriction enzyme. After fill-in, a unit (Eco RI/Hind III fragment) capable of expressing mouse dhfr cDNA under the control of UK promoter was isolated. Upon digestion with restriction enzyme, Apa LI was used to digest the region other than expression unit, thereby to make it possible to easily isolate the desired fragment. The resulting dhfr expression unit was ligated with pTY006 which had been previously digested with Xba I and subjected to fill-in, and then introduced into E. coli HB101. The colonies formed were screened. As the result, there was harvested a strain bearing the desired plasmid pTY007 in which the unit capable of expressing human ATIII under the control of SV40 promoter and the unit capable of expressing mouse dhfr under the control of UK promoter were inserted in the same direction.

Next, utilizing the fact that Xba I site in pTY007 is reproduced after fill-in and ligation, plasmid pTY008 in which dhfr expression unit had been inserted in a reversed direction was constructed. Firstly, pTY007 was digested with Xba I. After enzyme was inactivated by heating, ligation was performed. E. coli HBI0I and JMI09 were transformed with the reaction product, and the resulting colonies were analyzed. As the result, clone bearing pTY008 was harvested from the JMI09 colonies.

The restriction enzyme map of pTY007 and gene configuration are shown in Fig. 9. In Fig. 9, I: fragment containing about 340 bp region containing the region from Pvu II site (270) to Hind III (5I7I) of SV40; 2: about I.5 kb human ATIII cDNA which contains neither polyadenylation signal nor Poly(A) sequence and wherein Eco RI sites are reproduced at both termini; 3: about I.6 kb fragment containing the region (splicing junction) from Mbo I (47I0) to Mbo I (4I00) of SV40 and the region (polyadenylation signal) for Bgl I (2778) to Eco RI (I782) of SV40 downstream of the former region: 4: about 800 bp fragment containing promoter region of human urokinase; 5: about 740 bp fragment containing mouse dhfr cDNA; 6: fragment similar to 3, except that the polyadenylation signal portion contains only up to Pst I site (I988) of SV40 and Eco RI site at 3' end is reproduced; 7: pBR322-derived about I.84 kb fragment from Eco RI (436I) to Pvu II (2066).

[II] Introduction into animal cell

(i) Material and method

(I) Plasmid

pTY007: Plasmid in which DHFR transcription unit under the control of UK promoter and human ATIII transcription unit under the control of SV40 promoter have been arranged in the same direction.

pTY008: Plasmid in which human ATIII transcription unit under the control of SV40 promoter has been arranged downstream of DHFR transcription unit under the control of UK promoter in the inverted direction.

After preparation, the plasmids were purified by cesium chloride equilibrium density gradient centrifugation.

(2) Cell

CHO-KI cell-derived DHFR-deficient strain.

The strain was prepared and amplified according to the method described in Proc. Natl. Acad. Sci. (USA), 77, 42I6 (I980).

(3) Methotrexate (MTX)

(+) Amethopterin manufactured by Sigma Co. was dissolved in 0.82% (w/v) NaCl, 0.02% (w/v) $Na_2HPO_4$ and 0.56% (w/v) HEPES (Nakarai Tesque) to prepare 2 mM stock solution. The stock solution was added to medium in a desired concentration, which was provided for use.

(4) Medium and serum

MEM-α (containing ribonucleoside and deoxyribonucleoside) (Gibco).

MEM-α (containing neither ribonucleoside nor deoxyribonucleoside) (Gibco).

These media are abbreviated as MEM-α (w) and MEM-α (w/o), respectively. As serum, fetal calf serum (Mitsubishi Chemical Industry Co., Ltd., MH0I) was used after immobilization.

(5) Transfection of DNA and selection of transfectant

DXB-II cells subcultured in MEM-α (w) supplemented with I0% FCS were treated with trypsin (0.25% trypsin) to peel apart from a dish and I.5/I0⁵ cells were inoculated on a dish of I0 cm. Transfection was effected by the calcium phosphate method. Calcium phosphate precipitates were prepared using Cell Phect Transfection Kit of Pharmacia Fine Chemicals, Inc. Firstly, I0 μg of the expression plasmid was dissolved in 240 μl of sterile water. Then 240 μl of Solution A of the kit was added to the solution. After mixing them, the mixture was allowed to stand at room temperature for I0 minutes. Then 480 μl of Solution B was added to the mixture followed by allowing to stand at room temperature for I5 minutes (960 μl in total). The resulting calcium phosphate precipitates were immediately added to the cells. After culturing for 5.5 hours, the medium was removed. After washing, 3 ml of glycerol solution, of which composition is given below, was added.

$$50\% \text{ Glycerol} \qquad \char"3F 0 \text{ ml}$$

$$2 \times \text{HBS}^{*)} \qquad 50 \text{ ml}$$

$$H_2O \qquad 20 \text{ ml}$$

*) 2 x HBS (pH 7.1):  1.636% (w/v) NaCl, 1.118% (w/v) HEPES, 0.05 (w/v) $Na_2HPO_4$

After incubation at 37°C accurately for 2 minutes, the glycerol solution was removed. After washing and then culturing in MEM-α (w) containing l0% FCS for 3 days, the medium was exchanged with MEM-α (w/o) containing l0% FCS which was a selection medium. The medium was exchanged every 2 or 3 days. After culturing for about 2 weeks, colonies of the transfectants were formed. The transfectants were inoculated on a 96-wells plate in a ratio of 0.7 cell/well. Each well was microscopically examined and wells in which only one clony grew were selected. An amount of ATIII in the supernatant was assayed. The cell which gave the highest activity was scaled up and cloned again. The clone was subjected to DNA amplification with MTX.

(6) Amplification of the transfect4ed gene with MTX

The ATIII-producing cells obtained in (5) above were inoculated on MEM-α (w/o) containing l0% FCS and 5 nM MTX charged in a 6 wells plate in a concentration of 2 x l0$^5$ cells/well. When exchange of the medium was continued for every 3 other days, resistant strains appeared. When the cell count became sufficient in 2 to 4 weeks, the cells were subcultured in medium having a MTX concentration of next stage. As such, MTX concentration was gradually increased by 2 to 4 times each, starting from 5 nM of MTX concentration. MTX-resistant cells in each concentration were cultured in a 6-wells plate and an amount of ATIII in each supernatant was determined.

(7) Determination of amount of ATIII produced

ATIII gene-transfected CHO cells were suspended in MEM-α (purchased from Gibco) supplemented with l0% FCS followed by culturing in a 6-wells plate or a Petri dish of l0 cm in diameter. The medium was exchanged every 3 other days. After the cells reached saturation density (about l x l0$^6$ cells/well), the medium was exchanged followed by culturing for further 3 days. The culture supernatant was recovered and ATIII in the supernatant was assayed by ELISA (Stephens et al., Proc. Natl. Acad. Sci. USA, 84: 3886, l987). Furthermore, the cells were recovered by treating with 0.25% trypsin purchased from Sigma and the cells were counted.

(ii) Results

(l) ATIII production of early transfectant

Plasmids pTY007 and pTY008 were transfected to dhfr-deficient CHO cells. In order to select the cells having DNA encoding dhfr incorporated therein, transfectants were selected in nuceoside-free MEM-α (w/o) medium, as for l40 strains in which pTY007 had been transfected and 80 strains in which pTY008 had been transfected. The colonies formed in the medium were transferred to a 96-wells plate. When the cells became almost dense, the amount of ATIII in the supernatant (l00 μl/well) was determined by EIA.

There was a difference in production amounts between the cells transfected with pTY007 and the cells transfected with pTY008. With the cells transfected with pTY007, the strains which produced ATIII in an amount as high as more than l000 ng/ml but the ATIII production amount of the cell transfected with pTY008 was generally poor (Fig. l0).

(2) Increase in amount of ATIII produced by addition of MTX

Among l40 strains transfected with pTY007, 40 strains which provided high production amount of ATIII were treated with methotrexate (MTX), to attempt an increase in production amount by gene amplification.

Amounts of ATIII produced by representative MTX-resistant strains to 0 to 20 μM MTX are shown in Tables 5 and 6.

The production amount is expressed in terms of production amount per volume of culture broth (ng/ml/3 days) in Table 5 and in terms of production amount per cells (ng/l0$^6$ cells/3 days) in Table 6.

Table 5

Change in production amount of ATIII per volume of
culture broth of MTX-resistant strain (ng/ml/3 days)

| Clone No. | 0 nM | 5 nM | 20 nM | 50 nM | 150 nM | 400 nM | 1 μM | 2 μM | 5 μM | 20 μM |
|---|---|---|---|---|---|---|---|---|---|---|
| 18 | 262 | 1,720 | 2,820 | 4,440 | 6,000 | 8,210 | 10,700 | 10,200 | 3,130 | - |
| 24 | 398 | 1,550 | 2,730 | 4,580 | 8,300 | 8,740 | 6,530 | 9,280 | 9,510 | 4,380 |
| 36 | 439 | 1,240 | 1,410 | 523 | 917 | 677 | 6,220 | 7,610 | 5,010 | - |
| 55 | 150 | 154 | 931 | 898 | 2,190 | 3,830 | 5,180 | 4,650 | 7,300 | 3,310 |

EP 0 457 527 A1

Table 6

Change in production amount of ATIII per cell
of MIX-resistant strain (ng/$10^6$ cells/3 days)

| Clone No. | 0 nM | 5 nM | 20 nM | 50 nM | 150 nM | 400 nM | 1 μM | 2 μM | 5 μM | 20 μM |
|---|---|---|---|---|---|---|---|---|---|---|
| 18 | 88.8 | 734 | 1,270 | 2,540 | 2,350 | 4,740 | 5,090 | 7,180 | 3,520 | – |
| 24 | 213 | 835 | 1,380 | 3,140 | 4,900 | 5,620 | 4,210 | 11,300 | 4,760 | 3,200 |
| 36 | 184 | 715 | 922 | 305 | 566 | 595 | 5,650 | 4,820 | 6,770 | – |
| 55 | 65.3 | 91.2 | 466 | 643 | 1,650 | 3,730 | 4,750 | 3,520 | 6,620 | 2,220 |

Furthermore, with respect to each strain, the maximum production amount and degree of MTX resistance and also increment in production amount by MTX treatment are shown in Table 7.

## Table 7

### Comparison in the maximum production amount of ATIII after and prior to MTX treatment

| | Prior to MTX treatment | When resistant strain shows the maximum production amount | | |
|---|---|---|---|---|
| Clone No. | Production amount of ATIII per cell (ng/$10^6$/ 3 days) | Production amount of ATIII per cell (ng/$10^6$/ 3 days) | Concent- ration of MTX (µM) | Increment of pro- duction amount |
| 18 | 88.8 | 7,180 | 2.0 | 80.9 |
| 24 | 213 | 11,300 | 2.0 | 53.1 |
| 36 | 184 | 6,770 | 5.0 | 36.8 |
| 55 | 65.3 | 6,620 | 5.0 | 101.4 |

The production amount with the strains prior to MTX treatment was l00 to l000 ng/ml in terms of volume of culture broth and 20 to 600 ng/l0⁶ cells in terms of per cell. As a general tendency, as the degree of MTX resistance increases, the production amount increases; after here reached plates, the production amount decreased. The degree of MTX resistance to the maximum production varied depending on strain. While the maximum production amount of ATIII varied depending on kind of strain, it was l0 µg/ml/3 days per volume of culture broth and l0 µg/l0⁶ cells/3 days even per cell.

## Claims

1. A plasmid having i) a promoter capable of regulating the expression of a foreign protein in an animal cell and ii) a DNA encoding dihydrofolate reductase (DHFR) upstream of which urokinase (UK) promoter is ligated.

2. A plasmid having i) a DNA encoding a foreign protein upstream of which a promoter capable of regulating the expression of the protein in an animal cell is ligated and ii) a DNA encoding dihydrofolate reductase (DHFR) upstream of which an urokinase (UK) promoter is ligated.

3. An animal cell transformed with a plasmid according to claim 2.

4. An animal cell cotransformed with a plasmid having a DNA encoding a foreign protein upstream of which a promoter capable of regulating the expression of the protein in an animal cell and another plasmid having a DNA encoding dihydrofolate reductase (DHFR) upstream of which an urokinase (UK) promoter is ligated.

5. A process for producing a foreign protein which comprises culturing an animal cell according to claim 3 to express the foreign protein and recovering it.

6. A process for producing a foreign protein which comprises culturing an animal cell according to claim 4 to express the foreign protein and recovering it.

F I G. 1

# F I G. 2

SV40 E/P

m-PPA cDNA

# F I G. 3

# F I G. 4

# F I G. 5

THE CONSTRUCTION OF pTT08 AND pTT09

■ : SV40 DNA     ▨ : m-PPA cDNA     ▨ : UK PROMOTER

▦ : DHFR cDNA     ☐ : pBR 322 DNA

# F I G. 6

■ : SV40 DNA    ▨ : m-PPA cDNA    ▨ : UK PROMOTER

▦ : DHFR cDNA    □ : pBR 322 DNA

# F I G. 7

# FIG. 8

# FIG. 9

# F I G. IO

AMOUNT OF PRODUCED AT III
(ng/mℓ)

*) PRODUCING CLONE / TOTAL CLONE

□: DISTRIBUTION OF AT III — PRODUCING CLONES IN CHO CELLS
    TRANSFECTED WITH pTY007

▣: DISTRIBUTION OF AT III — PRODUCING CLONES IN CHO CELLS
    TRANSFECTED WITH pTY008

**European Patent Office**

# EUROPEAN SEARCH REPORT

Application Number

EP 91 30 4271

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-265 874 (THE GREEN CROSS CORPORATION)<br>* the whole document * | 1 | C12N15/85<br>C12N15/58<br>C07K15/00 |
| A | EP-A-281 246 (INVITRON CORPORATION)<br>* the whole document * | 1 | |
| A | NUCLEIC ACIDS RESEARCH.<br>vol. 16, no. 22, November 25, 1988, ARLINGTON, VIRGINIA US<br>pages 1069 - 9716;<br>VERDE, P. ET AL.: 'An upstream enhancer and a negative element in the 5' flanking region of the human urokinase plasminogen activator gene '<br>* the whole document * | 1 | |
| A | JOURNAL OF BIOLOGICAL CHEMISTRY.<br>vol. 263, no. 5, February 15, 1988, BALTIMORE US<br>pages 2460 - 2468;<br>NAKAGAWA, J. ET AL.: 'Transcriptional regulation of aPlasminogen Activator gene by cyclic AMP in a homologous cell-free system '<br>* the whole document * | 1 | **TECHNICAL FIELDS SEARCHED (Int. Cl.5)** |
| A | JOURNAL OF BIOLOGICAL CHEMISTRY.<br>vol. 262, no. 5, February 15, 1987, BALTIMORE US<br>pages 2339 - 2344;<br>O'CONNELL, M.L. ET AL.: 'Hormonal regulation of Tissue Plasminogen Activator secretion and mRNA levels in rat granulosa cells '<br>* the whole document * | 1 | C12N<br>C07K |
| A,P | MOLECULAR ENDOCRINOLOGY<br>vol. 4, no. 6, June 1990,<br>pages 940 - 946;<br>ROSSI, P. ET AL.: 'Follicle-Stimulating Hormone and cyclic AMP induce transcription from the Human Urokinase promoter in primary cultures of mouse Ser toli cells '<br>* the whole document * | 1 | |

The present search report has been drawn up for all claims

| Place of search | Date of completion of the search | Examiner |
|---|---|---|
| THE HAGUE | 21 AUGUST 1991 | CHAMBONNET F.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)